Europäisches Patentamt

European Patent Office

Office européen des brevets

(19)

(11) Veröffentlichungsnummer: **0 270 555**
**B1**

(12) **EUROPÄISCHE PATENTSCHRIFT**

(45) Veröffentlichungstag der Patentschrift: **02.01.91**

(51) Int. Cl.⁵: **A 61 F 5/01**

(21) Anmeldenummer: **87902074.1**

(22) Anmeldetag: **19.01.87**

(86) Internationale Anmeldenummer:
**PCT/EP87/00024**

(87) Internationale Veröffentlichungsnummer:
**WO 87/07498 17.12.87 Gazette 87/28**

(54) **SPRUNGGELENKPROTHESE.**

(30) Priorität: **03.06.86 DE 3618577**

(43) Veröffentlichungstag der Anmeldung:
**15.06.88 Patentblatt 88/24**

(45) Bekanntmachung des Hinweises auf die
Patenterteilung:
**02.01.91 Patentblatt 91/01**

(84) Benannte Vertragsstaaten:
**CH DE IT LI**

(56) Entgegenhaltungen:
**DE-A-3 228 753**
**US-A-4 289 122**
**US-A-4 316 454**

(73) Patentinhaber: **Biedermann, Lutz**
**Am Schäfersteig 8**
**D-7730 VS-Villingen (DE)**

(72) Erfinder: **Biedermann, Lutz**
**Am Schäfersteig 8**
**D-7730 VS-Villingen (DE)**

(74) Vertreter: **Prüfer, Lutz H., Dipl.-Phys.**
**Harthauser Strasse 25d**
**D-8000 München 90 (DE)**

Courier Press, Leamington Spa, England.

**Beschreibung**

Die Erfindung betrifft eine Sprunggelenkorthese mit einer den Mittelfuß und die Ferse wenigstens teilweise umfassenden Fußfassung und einer mit dieser über ein Gelenk verbundenen, den aufzunehmenden Unterschenkel wenigstens teilweise umfassenden Unterschenkelfassung.

Aus der US-PS 4 320 748 ist eine Fußschiene mit einer den Mittelfuß und die Ferse umfassenden Fußfassung und einer Unterschenkelfassung zur Stabilisierung von Knochenfrakturen bekannt. Die Unterschenkelfassung umfaßt die Unterschenkel muskulatur, damit über eine Muskelverspreizung der Unterschenkelmuskulatur eine Stabilisierung eintritt. An der Fußfassung ist ein Drehgelenk vorgesehen, welches lattenförmige Ansatzstücke aufweist, die über einen Klettverschluß mit einem entsprechenden Gegenstück an der Unterschenkelfassung in Verbindung bringbar sind. Eine Stabilisierung der Pro- Supinationsbewegung im unteren Sprunggelenk ist nicht vorgesehen.

Aus der DE-GM 86 03 420 ist eine Gelenkorthese für die Stützung und Führung des Sprunggelenkes aus zwei seitlichen Laschen, die über einen Mittelsteg miteinander verbunden sind, und bei der die Enden der Laschen gegebenenfalls durch eine Binde bzw. Klettverschlüsse am Fuß festlegbar sind, bekannt. Bei dieser weist ein U-förmiges Fußteil eine Fußplatte zur kippsteifen Halterung auf. Die Laschen werden in Knöchelhöhe jeweils durch ein Gelenkelement mit einem einstellbaren Schwenkbereich fortgesetzt. Das Gelenkelement ist in seiner Verlängerung mit einem Seitenteil verbunden.

Aus der DE-OS 25 16 945 ist es bekannt, eine orthopädische Vorrichtung aus thermisch verformbaren Kunststoffteilen zu bilden.

Aufgabe der Erfindung ist es, eine Sprunggelenkorthese der eingangs beschriebenen Art zu schaffen, mit der eine Stabilisierung des unteren Sprunggelenkes bei Bandinstabilität und Führung nach Bandverletzung erreicht wird, die Gelenkigkeit des oberen Sprunggelenkes erhalten bleibt, wobei eine Anpas-Sprunggelenkorthese an den jeweiligen Fuß auf einfache Weise möglich ist.

Diese Aufgabe wird durch eine Sprunggelenkorthese der eingangs beschriebenen Art mit den Merkmalen des kennzeichnenden Teiles des Patentanspruches 1 gelöst. Durch das Freilegen der Auftrittsfläche des Rückfußes hat der Benutzer der Orthese beim Fersenauftritt einen direkten Kontakt zum Schuh. Dies ist wichtig für die Rückmeldung zur Muskelkontrolle beim Sportler. Die Freigabe des Muskelbauches der Wadenmuskulatur bewirkt, daß die Wadenmuskeln nicht durch Kompression beeinträchtigt werden. Durch die wählbare Lage des Gelenkes relativ zur Fußfassung ist es möglich, die dem jeweiligen Fuß zugehörige Drehpunktlage für die Sprunggelenkorthese zu verwenden.

Weitere Merkmale und Zweckmäßigkeiten der Erfindung ergeben sich aus der Beschreibung eines Ausführungsbeispiels anhand der Figuren. Von den Figuren zeigen:

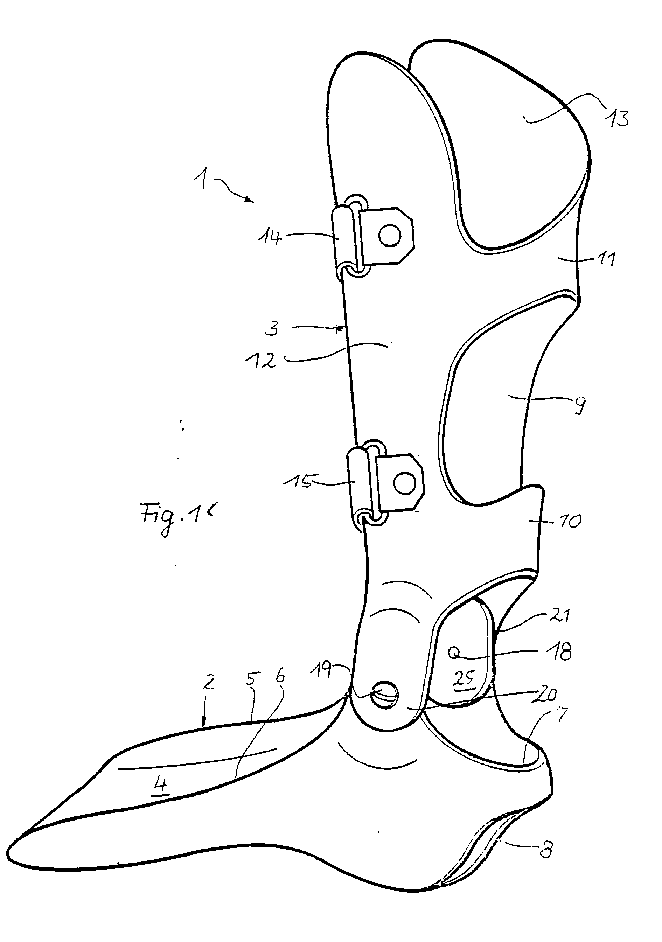

Fig. 1 eine perspektivische Seitenansicht einer Sprunggelenkorthese;

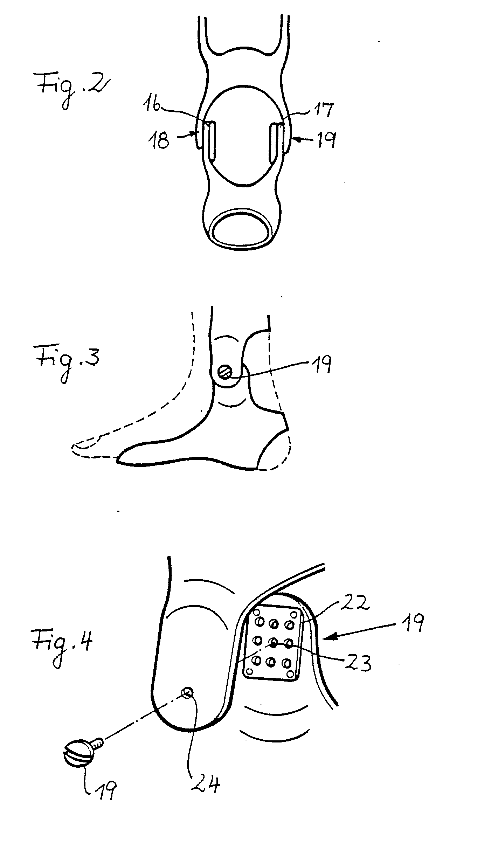

Fig. 2 eine perspektivische Teilansicht von vorn;

Fig. 3 eine Seitenansicht mit gestrichelt angedeutetem Fuß; und

Fig. 4 eine Explosionsdarstellung eines Gelenkes.

Die Sprunggelenkorthese 1 umfaßt eine Fußfassung 2 und eine Unterschenkelfassung 3.

Die Fußfassung 2 umfaßt den Mittelfuß schalenförmig und fixiert diesen, wobei zusätzlich das innere Längsgewölbe und das vordere Quergewölbe des Mittelfußes in die Unterstützung durch Umfassen durch einen entsprechenden Sohlenbereich 4 und daran angeformte seitliche Stützbereiche 5, 6 mit einbezogen werden. Im Bereich der Ferse gehen die beiden anatomisch geformten Stützbereiche in eine ausreichend hochgezogene Wand 7 über, die die aufzunehmende Ferse auf der Rückseite gut umschließt. An der Fersenaustrittsfläche ist eine Ausnehmung 8 vorgesehen, die so ausgebildet ist, daß sie die Auftrittsfläche des Rückfußes derart freilegt, daß der Benutzer der Orthese beim Fersenauftritt einen direkten Kontakt zum Schuh hat, wie dies am besten aus Fig. 3 ersichtlich ist.

Die Unterschenkelfassung 3 ist als eine den aufzunehmenden Unterschenkel umfassende Spange ausgebildet. Dabei reicht es je nach Anwendungszweck aus, den Unterschenkel im Hinblick auf die Höhe der Umfassung nur teilweise zu umfassen. Die gezeigte Ausführungsform ist so ausgebildet, daß sie den Unterschenkel in etwa einem Drittel seiner Höhe umfaßt.

Auf ihrer Rückseite ist die Unterschenkelfassung 3 so ausgebildet, daß an der dem Muskelbauch entsprechenden Stelle eine von dem darunterliegenden Bereich 11 begrenzte Ausnehmung 9 vorgesehen ist. Diese ist so ausgebildet, daß durch sie der Muskelbauch der Wadenmuskulatur frei beweglich offenliegt, so daß diese Muskeln nicht durch Kompression beeinträchtigt werden. Ferner umfaßt die Unterschenkelfassung mit den den Unterschenkel auf der Rückseite umgebenden Bereichen 10 und 11 einstückig ausgebildete seitliche Wangen 12, 13, die den Unterschenkel seitlich umfassen.

Auf seiner Vorderseite sind Klettbandverschlüsse 14, 15 vorgesehen, die ein festes Umfassen des Unterschenkels ermöglichen.

Die Fußfassung 2 weist in dem den Knöcheln des aufzunehmenden Fußes entsprechenden Bereich auf beiden Seiten sich nach oben erstreckende, Gelenkflächen bildende Ansätze 16, 17 zur Bildung von jeweiligen Gelenken 18, 19 auf. Die Unterschenkelfassung weist einstückig mit ihr ausgebildete, sich nach unten erstreckende, Gelenkflächen bildende Ansätze 20, 21 auf, die so geformt sind, daß sie zusammen mit dem jeweiligen unteren Ansatz 16, 17 ein Gelenk bilden.

Die Ansätze 16, 17 der Fußfassung weisen, wie am besten aus Fig. 4 ersichtlich ist, eine Lagerplatte 22 mit einer Mehrzahl von in vertikaler und horizontaler Richtung gegeneinander versetzten Löchern 23 auf, die sich durch den jeweiligen zugehörigen Ansatz hindurch erstrecken. Die Ansätze der Unterschenkelfassung weisen dagegen jeweils nur ein Loch 24 auf. Die Einstellung der Lage erfolgt dadurch, daß die die Gelenkachsen bildenden jeweiligen Schrauben in ein entsprechend gewähltes Loch 23 der Lagerplatte 22 eingeführt und dann verschraubt werden. Dadurch wird eine Verbindung erzeugt, die die Fußfassung und die Unterschenkelfassung um eine sich im wesentlichen durch die beiden Knöchel erstreckende Achse drehbar, ansonsten aber fest verbindet. Der an den Knöcheln anliegenden Innenseite ist jeweils eine Polsterung 25 zur Verbesserung der Stabilisierung des Fußes in der Orthese vorgesehen.

Die Fußfassung und die Unterschenkelfassung sind jeweils einstückig aus einem Hochleistungsverbundwerkstoff gefertigt.

Die Gelenke 18, 19 sind so ausgebildet, daß die winkelmäßige Bewegung zwischen Fußfassung und Unterschenkelbereich 3 und damit der Beugungs- und Streckwinkel im Umfang einstellbar begrenzt sind.

Abgesehen von der Bewegung um die Gelenkachse sind die Fußfassung und die Unterschenkelfassung starr miteinander verbunden, so daß eine exakte Fixierung der knöchernen Gelenkteile, nämlich Mittelfußknochen, Sprung- und Fersenbein einerseits und Waden- und Schienbein andererseits erreicht werden. Durch die Einstellbarkeit des mechanischen Drehpunktes der Gelenke in mehreren Ebenen ist eine exakte Justierung der externen Gelenkachse zur anatomischen Gelenkachse des oberen Sprunggelenkes zur Vermeidung von Inkongruenzen erreichbar. Das Sprunggelenk kann im Sinne der Extension und Flexion frei bewegbar werden. Dagegen wird die Bewegung des unteren Sprunggelenkes im Sinne der Pro- und Supination blockiert.

**Patentansprüche**

1. Sprunggelenkorthese mit einer den Mittelfuß und die Ferse wenigstens teilweise umfassenden Fußfassung (2) und einer mit dieser über ein Gelenk (18, 19) verbundenen, den aufzunehmenden Unterschenkel wenigstens teilweise umfassenden Unterschenkelfassung (3), dadurch gekennzeichnet, daß die Fußfassung (2) an der Fersenauftrittsfläche eine Ausnehmung (8) und die den Unterschenkel circulär umfassende Unterschenkelfassung (3) im Bereich der Wadenmuskulatur eine diese freigebende hintere Ausnehmung (9) aufweisen und daß die Lage des Drehpunktes des die Fußfassung (2) und die Unterschenkelfassung (3) verbindenden Gelenkes (18, 19) relativ zur Fußfassung (2) einstellbar ist.

2. Sprunggelenkorthese nach Anspruch 1, dadurch gekennzeichnet, daß die Fußfassung (2) eine Mehrzahl von in Längs- und Querrichtung zueinander versetzten Bohrungen (23) zur Verbindung mit einem durch eine Bohrung (24) der Unterschenkelfassung (3) gehenden Gelenkstiftes (19) aufweist.

3. Sprunggelenkorthese nach Anspruch 1 oder 2, dadurch gekennzeichnet, daß die Fußfassung (2) ein dem Fuß angepaßtes Längs- und Quergewölbe aufweist.

4. Sprunggelenkorthese nach einem der Ansprüche 1 bis 3, dadurch gekennzeichnet, daß das Gelenk (18, 19) Unterschenkelfassung (3) und Fußfassung (2) zum Stabilisieren der Pro-Supinationsbewegung starr miteinander verbindet.

5. Sprunggelenkorthese nach einem der Ansprüche 1 bis 4, dadurch gekennzeichnet, daß die Orthese aus Hochleistungsverbundwerkstoffen gebildet ist.

6. Sprunggelenkorthese nach einem der Ansprüche 1 bis 5, dadurch gekennzeichnet, daß der Beugungs- und Streckungswinkel einstellbar ist.

**Revendications**

1. Orthèse de l'articulation tibio-tarsienne comprenant une coque de pied (2) recouvrante, au moins en partie, le métatarse et le talon, ainsi qu'une coque de jambe (3) recouvrante, au moins en partie, la jambe à y recevoir et reliée à la coque de pied (2) par un assemblage articulé (18, 19), caractérisée en ce que ladite coque de pied (2) présente une découpure (8) à la surface de pose du talon pendant que ladite coque de jambe (3) recouvrante la jambe circulairement est pourvue d'une découpure (9) arrière dans la zone de la musculature du mollet, qui dégage la dernière, et en ce que la position du centre de rotation dudit assemblage articulé (18, 19) reliant la coque de pied (2) avec la coque de jambe (3) est ajustable relativement à la coque de pied (2).

2. Orthèse de l'articulation tibio-tarsienne selon la Revendication 1, caractérisée en ce que ladite coque de pied (2) comprend une pluralité de forures (23) échelonnées l'une à l'autre en sens longitudinal et travers pour la liaison au moyen d'une cheville de charnière (19) passante par une forure (24) dans la coque de jambe (3).

3. Orthèse de l'articulation tibio-tarsienne selon la Revendication 1 ou 2, caractérisée en ce que ladite coque de pied (2) présente une voûte longitudinale et transversale adaptée au pied.

4. Orthèse de l'articulation tibio-tarsienne selon quelconque des Revendications 1 à 3, caractérisée en ce que ledit assemblage articulé (18, 19) relie ladite coque de jambe (3) rigidement avec ladite coque de pied (2) d'une façon que le mouvement de pro-supination soit stabilisé.

5. Orthèse de l'articulation tibio-tarsienne selon quelconque des Revendications 1 à 4, caractérisée en ce que l'orthèse est constituée en matériau composite de qualité élevée.

6. Orthèse de l'articulation tibio-tarsienne selon quelconque des Revendications 1 à 5, caractérisée en ce que l'angle de flexion et d'extension est réglable.

## Claims

1. Ankle joint orthosis comprising a foot shell (2) enclosing, at least in part, the metatarsus and the heel, as well as a lower-leg shell (3) connected to said foot shell through a joint (18, 19) and enclosing, at least in part, the lower leg to be accomodated therein, characterized in that said foot shell (2) includes a cut-out (8) at the heel walking surface while said lower-leg shell (3) circularly enclosing the lower leg presents a rear cut-out (9) in the area of the muscles of the calf so as to leave these muscles uncovered, and that the position of the fulcrum of said joint (18, 19) interconnecting said foot shell (2) and said lower-leg shell (3) is adapted for adjustment relative to said foot shell (2).

2. Ankle joint orthosis according to Claim 1, characterized in that said foot shell (2) includes a plurality of bores (23) mutually offset in the longitudinal and the transverse directions for connection by means of a hinge pin (19) passing through a bore (24) of said lower-leg shell (3).

3. Ankle joint orthosis according to Claim 1 or 2, characterized in that said foot shell (2) is provided with a longitudinal and a transverse arch adapted to the foot.

4. Ankle joint orthosis according to any of Claims 1 to 3, characterized in that said joint (18, 19) rigidly interconnects said lower-leg shell (3) and said foot shell (2) so as to provide for a stabilization of the pro-supination movement.

5. Ankle joint orthosis according to any of Claims 1 to 4, characterized in that the orthosis is made of high-quality composite materials.

6. Ankle joint orthosis according to any of Claims 1 to 5, characterized in that the flexing and extending angle may be adjusted.

Fig. 1

Fig.2

16
17
18
19

Fig.3

19

Fig.4

22
19
23
24
19